# EUROPÄISCHE PATENTSCHRIFT

(11) **EP 2 229 699 B1**
(45) Veröffentlichungstag und Bekanntmachung des Hinweises auf die Patenterteilung: **23.05.2012**
(21) Anmeldenummer: 09701945.9
(22) Anmeldetag: 14.01.2009
(51) Int. Cl.: H01L 51/50, H01L 51/00, C07F 1/08, C07F 1/12, C07F 3/06, C07F 3/08, C07F 3/10, C07F 15/06

(54) **DITHIOLENÜBERGANGSMETALLKOMPLEXE UND ELEKTRONISCHE ODER OPTOELEKTRONISCHE BAUELEMENTE**
DITHIOL TRANSITION METAL COMPLEXES, AND ELECTRONIC OR OPTOELECTRONIC COMPONENTS
COMPLEXES DE DITHIOLÈNE-MÉTAUX DE TRANSITION, ET COMPOSANTS ÉLECTRONIQUES OU OPTOÉLECTRONIQUES

(30) Priorität: 15.01.2008 DE 102008004433
(43) Veröffentlichungstag der Anmeldung: 22.09.2010
(73) Patentinhaber: Novaled AG, 01307 Dresden (DE)
(72) Erfinder: ZEIKA, Olaf, 06727 Theissen (DE); BURGHART, Markus, 01326 Dresden (DE); HARTMANN, Horst, 01326 Dresden (DE); WILLMANN, Steffen, 01277 Dresden (DE)
(74) Vertreter: Bittner, Thomas L.
(86) Internationale Anmeldenummer: PCT/DE2009/000032
(87) Internationale Veröffentlichungsnummer: WO 2009/089821

(56) Entgegenhaltungen:
- US-A- 4 769 292
- US-B2- 7 074 500

## Beschreibung

Die vorliegende Erfindung betrifft S-verbrückte Übergangsmetalldithiolenkomplexe, deren Verwendung als Dotandenprecursor zur Dotierung eines organischen halbleitenden Matrixmaterials, als Ladungsinjektionsschicht, als Elektrodenmaterial, als Trägermaterial, als Matrixmaterial selbst oder als Speichermaterial in elektronischen oder optoelektronischen Bauelementen, organische halbleitende Matrixmaterialien sowie elektronische und optoelektronische Bauelemente.

Seit der Demonstration von organischen Leuchtdioden und Solarzellen [C.W. Tang et al., Appl. Phys. Lett. 51 (12), 913 (1987)] sind aus organischen Dünnschichten aufgebaute Bauelemente Gegenstand intensiver Forschung. Derartige Schichten besitzen vorteilhafte Eigenschaften für die genannten Anwendungen, wie z.B. effiziente Elektrolumineszenz für organische Leuchtdioden, hohe Absorptionskoeffizienten im Bereich des sichtbaren Lichtes für organische Solarzellen, preisgünstige Herstellung der Materialien und Fertigung der Bauelemente für einfachste elektronische Schaltungen, u.a. Kommerzielle Bedeutung hat bereits der Einsatz organischer Leuchtdioden für Displayanwendungen.

In organischen lichtemittierenden Dioden (OLEDs) wird die Eigenschaft von Materialien ausgenutzt, Licht zu emittieren, wenn durch das Anlegen einer Spannung geeignete Ladungsträger gebildet werden, die bei ihrer Rekombination angeregte Zustände bilden, die wiederum unter Emission von Licht in den Grundzustand übergehen. OLEDs stellen eine interessante Alternative zu Kathodenstrahlröhren und Flüssigkristalldisplays dar, da sie aufgrund ihrer sehr kompakten Bauweise und ihres niedrigen Stromverbrauchs zur Herstellung von Flachbildschirmen und Displays für mobile Anwendungen, wie Mobiltelefone, Armbanduhren, Notebooks, PDAs etc., geeignet sind.

Organische lichtemittierende Dioden (OLEDs) bestehen in der Regel aus verschiedenen Schichten organischer Materialien, wobei mindestens eine Schicht (Emissionsschicht) eine elektrolumineszente Substanz enthält, die durch Anlegung einer Spannung zur Emission von Licht gebracht werden kann (Tang, US 4,769,292). Hocheffiziente OLEDs sind z.B. in US 7,074,500 beschrieben.

Als organische Bauelemente gelten Bauelemente, die mindestens eine organische Halbleiterschicht enthalten. Die organischen Halbleiterschichten enthalten unter anderem organische Moleküle, so genante "kleine Moleküle", oder auch organische Polymere, wobei die organischen Moleküle und die organischen Polymere als Einzelschicht oder als Mischung mit anderen organischen (z.B. in US2005 0110009 beschrieben) oder anorganischen Materialien halbleiter- oder metallähnliche Eigenschaften besitzen.

Die Halbleiterbauelemente, die einen großen Anteil an anorganischen Halbleiterelementen bzw. Schichten haben und gleichzeitig eine oder mehrere organische Halbleiterschichten oder organische Halbleitermaterialien beinhalten, sind sogenannte anorganisch-organische Hybridbauelemente. Diese Hybridbauelemente sind im Rahmen der Erfindung auch als organische Bauelemente zu verstehen.

Es ist bekannt, organische Halbleiter durch Dotierung hinsichtlich ihrer elektrischen Eigenschaften, insbesondere ihrer elektrischen Leitfähigkeit, zu verändern, wie dies auch bei anorganischen Halbleitern, wie Siliciumhalbleitern, der Fall ist. Hierbei wird durch Erzeugung von Ladungsträgern im Matrixmaterial eine Erhöhung der zunächst recht niedrigen Leitfähigkeit sowie je nach Art des verwendeten Dotanden eine Veränderung im Fermi-Niveau des Halbleiters erreicht. Eine Dotierung führt hierbei zu einer Erhöhung der Leitfähigkeit von Ladungstransportschichten, wodurch ohmsche Verluste verringert werden, und zu einem verbesserten Übergang der Ladungsträger zwischen Kontakten und organischer Schicht. Die Dotierung im Leitfähigkeitssinne ist durch einen Ladungsübertrag vom Dotanden auf ein nahe liegendes Matrixmolekül gekennzeichnet (n-Dotierung, Elektronenleitfähigkeit erhöht), bzw. durch den Übertrag eines Elektrons von einem Matrixmolekül auf einen nahe liegenden Dotanden (p-Dotierung, Löcherleitfähigkeit erhöht). Der Ladungsübertrag kann unvollständig oder vollständig erfolgen und lässt sich z.B. durch die Interpretation von Schwingungsbanden einer FTIR (fourier-transformed infrared-spectroscopy) Messung bestimmen.

Die bisher verwendeten anorganischen Dotanden, wie Alkali- oder Erdalkalimetalle (z.B. Cäsium) oder Lewis-Säuren (z.B. FeC13), sind bei organischen Matrixmaterialien aufgrund ihrer hohen Diffusionskoeffizienten meist nachteilig, da die Funktion und Stabilität der elektronischen Bauelemente beeinträchtigt wird. Mit diesen anorganischen Dotanden sind weiterhin Schwierigkeiten in der Produktion verbunden, da sie in der Regel einen hohen Dampfdruck bei Raumtemperatur aufweisen und in Vakuumprozessen die Produktionsanlagen kontaminieren können. Insbesondere Alkali- und Erdalkalimetalle haben den weiteren Nachteil, dass ihre Verwendung durch ihre starke Reaktivität mit Wasser und Luft erschwert ist.

Von besonderer Bedeutung ist bei üblichen organischen lichtemittierenden Dioden die Leitfähigkeit der Schichten, aus denen die OLED aufgebaut ist, insbesondere der dotierten Schichten. Die Leitfähigkeit einer Schicht kann bestimmt werden, indem auf ein Substrat zwei Kontakte in einem Abstand voneinander aufgebracht werden. Auf dieses Substrat wird dann die zu prüfende Schicht aufgebracht. Bei Anlegen einer Spannung fließt Strom durch die Probe. Mit Hilfe des Ohm'schen Gesetztes und der Geometrie der Probe kann die Leitfähigkeit bestimmt werden. Bei Betriebstemperatur eines elektronischen oder optoelektronischen Bauelements, das die dotierte Schicht beinhaltet, soll die Leitfähigkeit der dotierten Schicht die Leitfähigkeit der undotierten Schicht übertreffen. Undotierte Schichten weisen im Allgemeinen Leitfähigkeiten von kleiner 10⁻⁸ S/cm, meistens von kleiner als 10⁻¹⁰ S/cm auf, während dotierte Schichten häufig Leitfähigkeiten im Bereich von 10⁻⁸ S/cm, bevorzugter größer als 10⁻⁶ S/cm, noch bevorzugter größer als 10⁻⁵ S/cm aufweisen.

Die typische Struktur einer Standard-OLED ist dem Fachmann bekannt, sie kann wie folgt aussehen:
1. Träger, Substrat, z.B. Glas
2. Elektrode, löcherinjizierend (Anode = Pluspol), vorzugsweise transparent, z.B. Indium-Zinn-Oxid (TTO)
3. Löcherinjektionsschicht, z.B. CuPc (Kupfer-Phthalocyanin), oder Starburst-Derivate,
4. Löchertransportschicht, z.B. TPD (Triphenyldiamin und Derivate),
5. löcherseitige Blockschicht, um Exzitonendiffusion aus der Emissionsschicht-zu verhindern und Ladungsträger-Leckage aus der Emissionsschicht zu verhindern, z.B. alpha-NPB (Bis-naphtyl-phenylamino-biphenyl),
6. lichtemittierende Schicht oder System von mehreren zur Lichtemission beitragenden Schichten, z.B. CBP (Carbazol-Derivate) mit Emitterbeimischung (z.B. phosphoreszenter Triplett-Emitter Iridium-tris-phenylpyridin Ir(ppy)3) oder Alq3 (Tris-quinolinato-aluminium) gemischt mit Emittermolekülen (z.B. fluoreszenter Singlett-Emitter Qumarin),
7. elektronenseitige Blockschicht, um Exzitonendiffusion aus der Emissionsschicht zu verhindern und Ladungsträger-Leckage aus der Emissionsschicht zu verhindern, z.B. BCP (Bathocuproine),
8. Elektronentransportschicht, z.B. Alq3 (Tris-quinolinato-aluminium),
9. Elektroneninjektionsschicht, z.B. anorganisches Lithiumfluorid (LiF),
10. Elektrode, meist ein Metall mit niedriger Austrittsarbeit, elektroneninjizierend (Kathode = Minuspol), z.B. Aluminium.

Es können natürlich Schichten weggelassen werden, oder eine Schicht (respektive ein Material) kann mehrere Eigenschaften übernehmen, z.B. können die Schichten 3 und 4, 4 und 5, 3-5 zusammengefasst werden, bzw. die Schichten 7 und 8, 8 und 9, und 7-9 zusammengefasst werden. Weiter Möglichkeiten sehen die Mischung der Substanz aus Schicht 9 in die Schicht 8 vor etc..

Die reflektierende Elektrode und die organischen Schichten werden normalerweise durch thermische Vakuum-Verdampfung auf einem Substrat abgeschieden. Die Elektrode kann auch gesputtert werden. Unter anderem können die organischen Schichten aus Lösungsmitteln hergestellt werden, wie z.B. durch Spin-coating und Ink-jet-Drucken.

Dieser Aufbau beschreibt den nicht-invertierten (Anode auf dem Substrat), substratseitig emittierenden (bottom-emission) Aufbau einer OLED. Es gibt verschiedene Konzepte, vom Substrat weg emittierende OLEDs zu beschreiben (siehe Referenzen in DE102 15 210.1). Allen ist gemein, dass dann die substratseitige Elektrode (im nicht-invertierten Fall die Anode) reflektierend (oder transparent für eine durchsichtige OLED) ist und die Deckelektrode (semi-)transparent ausgeführt ist. Üblicherweise ist dies mit Leistungsparametereinbußen verbunden.

Wenn die Reihenfolge der Schichten invertiert wird (Kathode auf Substrat), spricht man von invertierten OLEDs (siehe Referenzen in DE101 35 513.0). Auch hierbei ist ohne spezielle Maßnahmen mit Leistungseinbußen zu rechnen.

Übliche organische Bauelemente sind auch OTFT, OSC, Speciher.

Der Aufbau von organischen Solarzellen ist dem Fachmann bekannt, siehe EP1861886 und EP1859494.

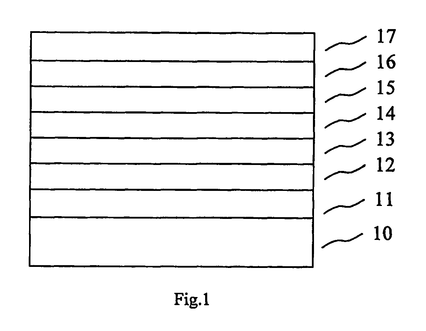

Die beigefügte Figur 1 zeigt einen Schnitt durch eine typische organische Solarzelle.

Figur 1 zeigt eine typische Schichtstruktur einer organischen Solarzelle im Querschnitt. Auf einem Substrat 10 sind die Schichten in der folgenden Reihenfolge aufgebaut: Anode 11, p-dotierte Löchertransportschicht 12, nicht-dotierte Löchertransportschicht 13, die auch photoaktiv sein kann, photoaktive Schicht 14, Elektronentransportschicht 15, die auch photoaktiv sein kann, n-dotierte Elektronentransportschicht 16 und Kathode 17. Weitere Schichtaufbaustrukturen für organische Solarzellen sind einem Fachmann ebenfalls bekannt. Zum Beispiel könnte anstelle der n-dotierten Elektronentransportschicht 16 eine dünne Pufferschicht verwendet werden. Zwei oder mehr Schichten können vereinigt sein, sobald kombinierte Eigenschaften vorhanden sind. Mehr Schichten, wie, z.B. Löcher- und auch Elektroneninjektionsschichten können vorhanden sein.

Organische Speicherbauelemente sind weit bekannt, die durch viele unterschiedliche Mechanismen funktionieren. Unter dessen gibt es Speicher, die durch die folgenden Mechanismen funktionieren: FE-RAM, elektrischer Durchbruch (Electrical breakdown), Ladungstopf (charge retention), und andere. Organische Speicher mit dotierten Schichten sind auch in der Patentanmeldung DE102007019260.8 beschrieben.

Der vorliegenden Erfindung liegt die Aufgabe zu Grunde, die Nachteile des Stands der Technik zu überwinden und insbesondere Verbindungen bereitzustellen, die zu verbesserten organischen halbleitenden Matrixmaterialien, Ladungsinjektionsschichten, Matrixmaterialien selbst, Elektrodenmaterialien, insbesondere in elektronischen oder optoelektronischen Bauelementen, führen. Vorzugsweise sollen die Verbindungen ausreichend hohe Reduktionspotenziale aufweisen, ohne störende Einflüsse auf das Matrixmaterial sein und eine wirksame Erhöhung der Ladungsträgeranzahl im Matrixmaterial bereitstellen und vergleichsweise einfach handhabbar sein. Schließlich soll bei Einsatz als Dotand die Leitfähigkeit einer dotierten Schicht beträchtlich verbessert werden.

Weitere Aufgaben der vorliegenden Erfindung liegen in der Bereitstellung von organischen halbleitenden Materialien und von elektronischen Bauelementen oder optoelektronischen Bauelementen, in denen die offenbarten Übergangsmetallkomplexverbindungen verwendet werden können.

Die erste Aufgabe wird gelöst durch einen Übergangsmetallkomplex, der die folgenden Strukturen (4), (5), (6) oder (7) aufweist 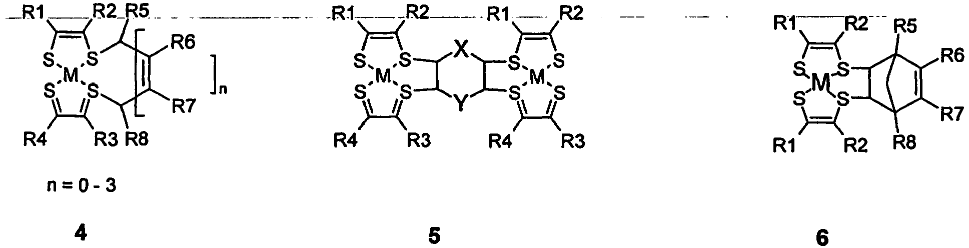 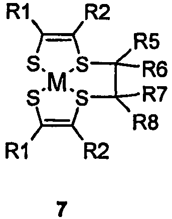 wobei M ein Übergangsmetall ist, das ausgewählt ist aus Cu, Au, Co, Zn, Cd und Hg;
R1-R4 unabhängig ausgewählt sind aus Wasserstoff; Halogen; Pseudohalogen; CN; NO₂; COR mit R = Alkyl, Alkenyl, Aryl oder Heteroaryl; geradkettigem, verzweigtem oder cyclischem, substituiertem oder unsubstituiertem Alkyl und Alkenyl; und substituiertem oder unsubstituiertem Aryl, Diaryl, Triaryl, Heteroaryl, Diheteroaryl oder Triheteroaryl
R5-R8 unabhängig ausgewählt sind aus Wasserstoff Alkyl, Alkenyl, Heteroalkyl, Aryl, Heteroaryl, oder R5, R6, R7 und/oder R8 über eine oder mehrere Brücken miteinander verbunden sind; und

X und Y unabhängig voneinander ausgewählt sind aus O, S, NR, Alkyl, Alkenyl, Heteroalkyl, Heteroalkenyl, Aryl, Heteroaryl, wobei auch eines von X und Y nicht vorhanden sein kann zur Ausbildung einer einfach verbrückten Struktur (5).

Bevorzugt ist dabei, dass R1-R4 unabhängig ausgewählt sind aus geradkettigem, verzweigtem oder cyclischem, substituiertem oder unsubstituiertem Alkyl und Alkenyl, vorzugsweise perhalogeniertem Alkyl

Auch wird vorgeschlagen, dass R5-R8 unabhängig ausgewählt sind aus verzweigtem, cyclischem, substituiertem oder unsubstituiertem Alkyl, Alkenyl, Heteroalkyl, substituiertem und unsubstituiertem Aryl und Heteroaryl, Diaryl, Diheteroaryl, Triaryl und Triheteroaryl.

Erfindungsgemäß ist ferner eine Verwendung eines Übergangsmetallkomplexes als Dotandenprecursor zur Dotierung und Photodotierung eines organischen halbleitenden Matrixmaterials, als Blockermaterial, als Ladungsinjektionsschicht, als Elektrodenmaterial, als Transportschicht, als Matrixmaterial selbst oder als Speichermaterial in elektronischen oder optoelektronischen Bauelementen, wobei der Übergangsmetallkomplex die folgenden Strukturen (4), (5), (6) oder (7) aufweist, 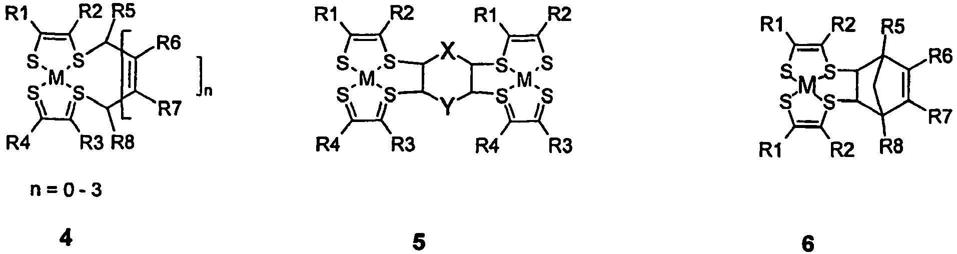 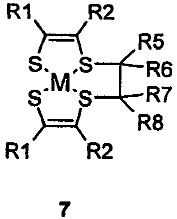 wobei M ein Übergangsmetall ist, das ausgewählt ist aus Ni, Pd, Pt, Cu, Au, Co, Zn, Cd und Hg;
R1-R4 unabhängig ausgewählt sind aus Wasserstoff; Halogen; Pseudohalogen; CN; NO₂; COR mit R = Alkyl, Alkenyl, Aryl oder Heteroaryl; geradkettigem, verzweigtem oder cyclischem, substituiertem oder unsubstituiertem Alkyl und Alkenyl; und substituiertem oder unsubstituiertem Aryl, Diaryl, Triaryl, Heteroaryl, Diheteroaryl oder Triheteroaryl
R5-R8 unabhängig ausgewählt sind aus Wasserstoff Alkyl, Alkenyl, Heteroalkyl, Aryl, Heteroaryl, oder R5, R6, R7 und/oder R8 über eine oder mehrere Brücken miteinander verbunden sind; und
X und Y unabhängig voneinander ausgewählt sind aus O, S, NR, Alkyl, Alkenyl, Heteroalkyl, Heteroalkenyl, Aryl, Heteroaryl, wobei auch eines von X und Y nicht vorhanden sein kann zur Ausbildung einer einfach verbrückten Struktur (5).

Dabei ist bevorzugt, dass R1-R4 unabhängig ausgewählt sind aus geradkettigem, verzweigtem oder cyclischem, substituiertem oder unsubstituiertem Alkyl und Alkenyl, vorzugsweise perhalogeniertern Alkyl.

Auch erfindungsgemäß ist ein organisches halbleitendes Material enthaltend zumindest eine organische Matrixverbindung und einen Dotandenprecursor, dadurch gekennzeichnet, dass als Dotandenprecursor ein erfindungsgemäßer Übergangsmetallkomplex verwendet wird.

Bevorzugt ist, dass das molare Dotierungsverhältnis von Dotandenprecursor zum Matrixmolekül bzw. das Dotierungsverhältnis von Dotandenprecursor zu monomeren Einheiten eines polymeren Matrixmoleküls zwischen 20:1 und 1:100000, bevorzugt 10:1 1 und 1:1000, besonders bevorzugt 1:1 1 und 1:100 beträgt.

Ebenfalls erfindungsgemäß ist ein elektronisches oder optoelektronisches Bauelement mit einem elektronisch funktionell wirksamen Bereich, dadurch gekennzeichnet, dass als elektronisch wirksamer Bereich zumindest ein erfindungsgemäßer Übergangsmetallkomplex verwendet wird.

Dabei ist bevorzugt, dass der elektronisch wirksame Bereich ein organisches halbleitendes Material aufweist, welches mit zumindest einem erfindungsgemäßen Übergangsmetallkomplex dotiert ist.

Das elektronisches oder optoelektronische Bauelement kann in Form einer organischen licht-emittierenden Diode, einer organischen Laserdiode, einer organischen Solarzelle, eines organischen Transistors, eines organischen Speicherbausteins, einer organischen Gleichrichterdiode, einer Photovoltaikzelle oder eines Photoleiters vorliegen.

Überraschender Weise wurde festgestellt, dass die erfindungsgemäßen Übergangsmetallkomplexe gegenüber herkömmlichen Dithiolenkomplexen eine erhöhte Stabilität gegenüber Wasser (Luftfeuchtigkeit) oder Sauerstoff zeigen, insbesondere eine ausgezeichnete Lagerstabilität. Mittels der erfindungsgemäßen Dithiolenkomplexe können extrem starke Akzeptorverbindungen dargestellt und stabilisiert werden, die bei entsprechender Verwendung zu verbesserten organischen halbleitenden Matrixmaterialien, Ladungsinjektionsschichten, Matrixmaterialien selbst, Elektrodenmaterialien, insbesondere in elektronischen oder optoelektronsichen Bauelementen führen. Durch die Addukt-Bildung aus Übergangsmetalldithiolen und Olefin kann die Sublimationstemperatur der Verbindung in geeigneter Weise verändert werden. Insbesondere eignen sich die erfindungsgemäßen Komplexe als lichtempfindliche Dotandenprecursor zur Dotierung und Photodotierung eines organischen halbleitenden Matrixmaterials, wobei auch eine Maskierung, d.h. eine Strukturierung mittels Licht, vorteilhaft verwendet werden kann.

Die erfindungsgemäßen Übergangsmetallkomplexe dienen als Dotandenprecursor und können mittels elektromagnetischer Strahlung, insbesondere durch Bestrahlung mit sichtbarem oder nahem UV-Licht oder thermisch oder elektrochemisch, in das jeweilige Olefin und den Dithiolenkomplex gespalten werden. Diese Spaltung kann während des Aufdampfvorganges oder in der Schicht erfolgen.

Sichtbares Licht ist elektromagnetische Strahlung mit einer Wellenlänge zwischen ungefähr 380 und 750 nm. UV-Licht hat einen Wellenlängebereich von ca. 10 nm bis ca. 400 nm, wobei der Bereich zwischen 380 nm und 400 nm eine Überlappung ist. Hier ist insbesondere der Bereich über ca. 200 nm interessant, weil Licht mit kürzerer Wellenlänge von typischen Substratmaterialien (Quarz, Glass, Plastik) sehr stark absorbiert wird.

Zur Aktivierung der Dotandenprecursor ist sichtbares Licht des UV/Vis-Bereichs von etwa 200 bis 800 nm, bevorzugt 300-520 nm, besonders bevorzugt 350-450 nm geeignet.

Als Zentralatom "M" können Übergangsmetalle eingesetzt werden, die quadratisch planare Dithiolen-Komplexe bilden können. Bezüglich der Reste R1-R4 werden besonders bevorzugt geradkettige, verzweigte oder cyclische, insbesondere perhalogenierte, vorzugsweise perfluorierte Alkyle, Alkenyle, Aryle oder Heteroaryle eingesetzt. Entsprechende Verbindungen können auch mit Akzeptorgruppen, wie CN, NO₂, COR mit R= Alkyl, Alkenyl, Aryl oder Heteroaryl substituiert sein.

Für die Reste R5-R8 sind beispielsweise Alkoxy, Phenoxy, Dialkylamin, Diarylamin, Diheteroarylamin, Monoalkylamin, Monoarylamin, Monoheteroarylamin, Piperidin, Morpholin, etc., einsetzbar.

Für X und Y können auch teilweise oder vollständig hydrierte Aryl- oder Heteroarylverbindungen eingesetzt werden. Eines der Elemente X oder Y kann auch nicht vorhanden sein, so dass eine einfach verbrückte Struktur resultiert.

Es ist ebenfalls vorstellbar, dass die einen oder mehreren Doppelbindungen im Übergangsmetallkomplex-Addukt teilweise oder vollständig gesättigt ist bzw. sind. Besonders bevorzugt ist n in Struktur (4) 1, 2 oder 3.

Erfindungsgemäße Übergangsmetallkomplexe der Struktur (6) können beispielhaft gemäß dem folgenden Schema hergestellt werden:

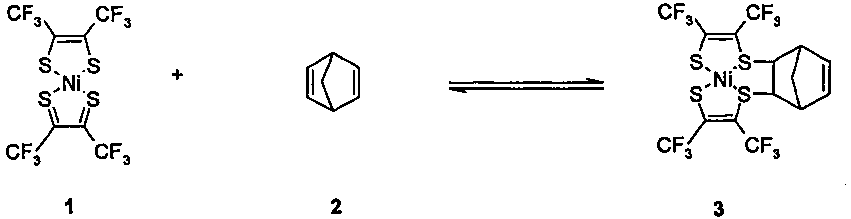

Das heißt, entsprechende S-verbrückte Übergangsmetalldithiolene lassen sich aus Übergangsmetalldithiolenen und Olefinen durch Cycloaddition darstellen, siehe K. Wang., E. I. Stiefel, Science (2001) 291, 106; W. E. Geiger, Inorg. Chem. (2002) 41, 136; R. M. Wing et al., JACS (1970) 92, 1935; G. N. Schrauzer, V. P. Mayweg, JACS (1965) 87, 1483; D. J. Harrison et al., JACS (2006) 128, 11026; W. E. Geiger et al., Inorg. Chem. (2001) 40, 2472. Veranschaulichend ist in der oben gezeigten Reaktionsgleichung die Umsetzung von Norbornadien 2 mit einem Nickeldithiolat 1 verdeutlicht, um das Addukt 3 zu bilden, welches mittels elektromagnetischer Strahlung in die Ausgangsstoffe aufgespalten werden kann.

Ebenfalls ist es denkbar, verschiedene Butadiene zur Adduktbildung einzusetzen, wie es von A. Herman, R M. Wing, J. Organometallic Chem. (1973) 63, 441, beschrieben wird.

### Darstellung von Bis(1,2-di(trifluormethyl)1,2-ethylendithiolato)cobalt(0)-Norbornadien-Addukt

Zu einer Lösung von 730 mg Cobaltdithiolat in 20 ml trockenem Chloroform wurden binnen 5 Minuten unter Argon 2 ml Norbornadien hinzugefügt und das dunkle Reaktionsgemisch 2 Stunden bei Raumtemperatur gerührt. Das ausgefallene mikrokristalline Produkt wurde abgesaugt und mit etwa 3 ml Petrolether (40/60) gewaschen. Es folgte eine abschließende Trocknung an der Luft. Die Ausbeute betrug 356 mg (41%) als braunes Pulver.

### Darstellung von Bis(1-heptafluor-iso-prolpyl-1,2-ethendithiolato)nickel(0)-Norbornadien-Addukt

1,5 g Ni-1-H-Dithiolat (unsymmetrisch) wurde in 30 ml trockenem Chloroform gelöst und diese Lösung mikrofiltriert (0,45 µm). Zu dieser tiefvioletten Lösung wurde unter Argon innerhalb von 15 Minuten eine Lösung aus 0,8 ml Norbornadien in 1 ml CHCl₃ zugetropft. Es wurde bei Raumtemperatur für 2 Stunden nachgerührt und die entstandene Kristallsuspension über Nacht in den Kühlschrank gestellt. Schließlich wurde abgesaugt und mit 2 ml Petrolether gewaschen. Anschließend erfolgte eine Trocknung an Luft. Die Ausbeute betrug 288 mg (17%) als hellgrünes mikrokristallines Pulver.

### Dotierung

Als p-dotierbare Matrixmaterialen können unter anderem Phthalocyaninkomplexe, beispielsweise des Zn (ZnPc), Cu (CuPc), Ni (NiPc) oder anderer Metalle, wobei der Phthalocyaninligand auch substituiert sein kann, eingesetzt werden. Auch andere Metallkomplexe von Naphtocyaninen und Porphyrinen können gegebenenfalls eingesetzt werden. Weiterhin können als Matrixmaterial auch arylierte oder heteroarylierte Amine bzw. Benzidinderivate eingesetzt werden, die substituiert oder unsubstituiert sein können, insbesondere auch Spiro-verknüpfte, beispielsweise TPD, α-NPD, TDATA, Spiro-TTB. Insbesondere können α-NPD und Spiro-TTB als Matrixmaterial eingesetzt werden.

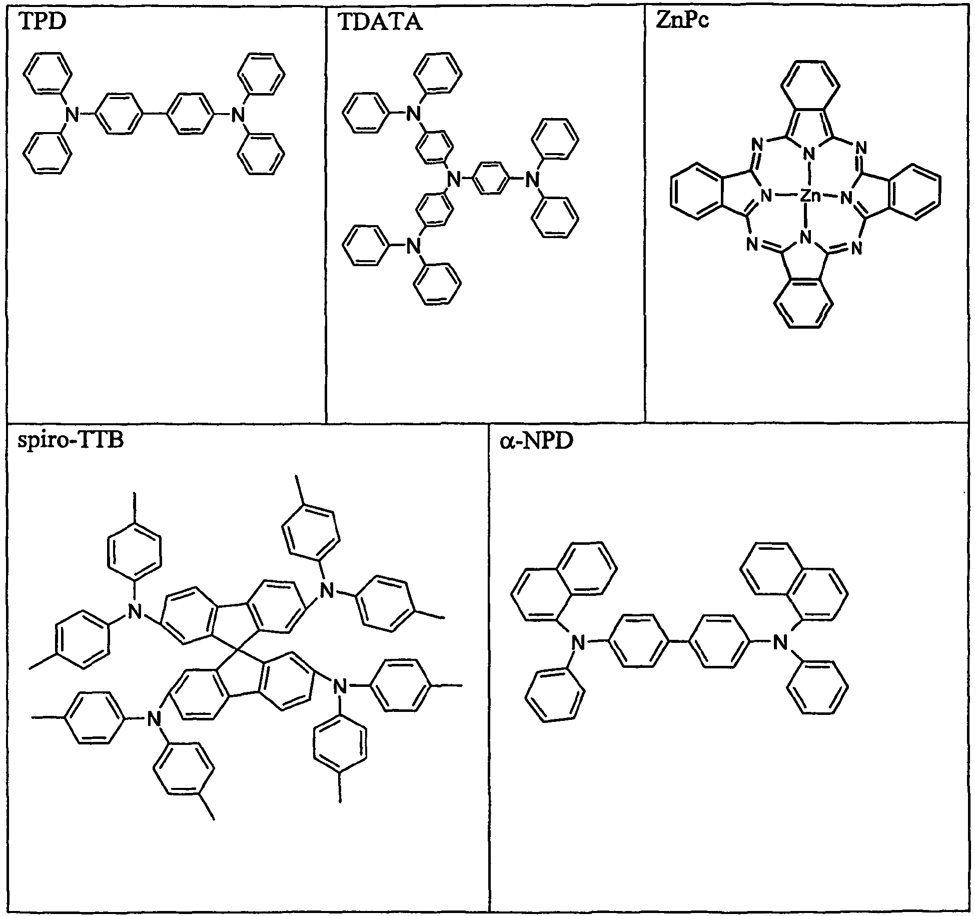

Als Matrixmaterial können neben polyaromatischen Kohlenwasserstoffen auch Heteroaromaten wie insbesondere Imidazol-, Thiophen, Thiazolderivate, Heterotriphenylene aber auch andere eingesetzt werden, gegebenenfalls auch dimere, oligomere bzw. polymere Heteroaromaten. Die Heteroaromaten sind vorzugsweise substituiert, insbesondere Aryl-substituiert, beispielsweise Phenyl- oder Naphthyl-substituiert. Sie können auch als Spiroverbindungen vorliegen. Insbesondere können obenstehende Verbindungen als Matrixmaterial eingesetzt werden. Es versteht sich, dass die genannten Matrixmaterialien auch untereinander oder mit anderen Materialien gemischt im Rahmen der Erfindung einsetzbar sind. Es versteht sich, dass auch geeignete andere organische Matrixmaterialien verwendet werden können, die halbleitende Eigenschaften aufweisen.

### Dotierungskonzentration

Vorzugsweise liegt der Dotand bzw. Dotandenprecursor in einer Dotierungskonzentration von ≤ 1:1 zu dem Matrixmolekül bzw. der monomeren Einheit eines polymeren Matrixmoleküls vor, vorzugsweise in einer Dotierungskonzentration von 1:2 oder kleiner, besonders bevorzugt von 1:5 oder kleiner oder 1:10 oder kleiner. Die Dotierungskonzentration kann sich in dem Bereich von 1:1 bis 1:100.000, insbesondere in dem Bereich von 1:5 bis 10.000 oder 1:10 bis 1.000 betragen, beispielsweise in dem Bereich von 1:10 bis 1:100 oder 1:25 bis 1:50, ohne hierauf beschränkt zu sein.

### Durchführung der Dotierung

Die Dotierung des jeweiligen Matrixmaterials mit den erfindungsgemäßen Verbindungen kann durch eines oder eine Kombination der folgenden Verfahren erfolgen:
a) Mischverdampfung im Vakuum mit einer Quelle für das Matrixmaterial und einer für den Dotandenprecursor.
b) Sequentielles Deponieren des Matrixmaterials und des p-Dotandenprecursors bzw. Dotanden auf einem Substrat mit anschliessender Eindiffusion des Dotanden bzw. Precursor, insbesondere durch thermische Behandlung.
c) Dotierung einer Matrixschicht durch eine Lösung von p-Dotandeprecursorn mit anschliessendem Verdampfen des Lösungsmittels, insbesondere durch thermische Behandlung.
d) Oberflächendotierung einer Matrixmaterialschicht durch eine oberflächlich aufgebrachte Schicht von Dotandenprecursorn.
e) Herstellung einer Lösung von Matrixmolekülen und Dotandenprecursorn und anschließende Herstellung einer Schicht aus dieser Lösung mittels konventioneller Methoden wie beispielsweise Verdampfen des Lösungsmittels oder Aufschleudern

Die Dotierung kann derart erfolgen, dass der Dotand aus der Precursor-Verbindung heraus verdampft wird, die beim Erhitzen und/oder Bestrahlen den Dotanden freisetzt. Durch eine Bestrahlung kann im wesentlichen die zur Verdampfung notwendige Wärme bereitgestellt werden, es kann auch gezielt in bestimmte Banden der zu verdampfenden Verbindungen bzw. Precursor oder Verbindungskomplexe wie Charge-Transfer-Komplexe eingestrahlt werden, um beispielsweise durch Überführung in angeregte Zustände die Verdampfung der Verbindungen durch Dissoziation der Komplexe zu erleichtern. Der Komplex kann aber insbesondere auch ausreichend stabil sein, um unter den gegebenen Bedingungen undissoziiert zu verdampfen oder auf das Substrat aufgebracht zu werden. Es versteht sich, dass auch andere geeignete Verfahren zur Durchführung der Dotierung eingesetzt werden können.

Auf diese Weise können somit p-dotierte Schichten von organischen Halbleitern hergestellt werden, die vielfältig einsetzbar sind.

### Halbleitende Schicht

Mittels der erfindungsgemäßen Übergangsmetallkomplexverbindungen können halbleitende Schichten erzeugt werden, die gegebenenfalls eher linienförmig ausgebildet sind, wie z.B. als Leitfähigkeitspfade, Kontakte oder dergleichen. Ebenso ist es möglich, in einer halbleitenden Schicht Leitfähigkeitspfade, Kontakte oder andere leitfähige Strukturen dadurch zu erzeugen, dass die oben genannte Behandlung der Schicht mit elektromagnetischer Strahlung nur lokal vorgenommen wird, wobei sich die leitfähigen Strukturen aus der Menge der bestrahlten Gebiete ergeben. Insbesondere kann die verbleibende Menge der nichtbestrahlten Schichtbereiche als Isolation für die bestrahlten Bereiche dienen. Die Übergangsmetallkomplexe können hierbei als p-Dotanden bzw. Dotandenprecursorn zusammen mit einer anderen Verbindung, die als Matrixmaterial fungieren kann, eingesetzt werden, wobei das Dotierungsverhältns 1 : 1 oder kleiner sein kann. Der verwendete Dotand bzw. Dotandenprecursor kann zu der jeweils anderen Verbindung bzw. Komponente aber auch in höheren Anteilen vorliegen, so dass das Verhältnis Dotand bzw. Precursor : Verbindung im Verhältnis > 1 : 1 liegen kann, beispielsweise im Verhältnis ≥ 2 : 1, ≥ 5 : 1, ≥ 10 : 1 oder > 20 : 1 oder höher. Die jeweils andere Komponente kann eine solche sein, wie sie als Matrixmaterial im Falle der Herstellung dotierter Schichten eingesetzt werden kann, ohne hierauf beschränkt zu sein. Gegebenenfalls kann der verwendete Dotand bzw. Precursor auch im wesentlichen in reiner Form vorliegen, beispielsweise als reine Schicht.

Der einen Dotanden bzw. Precursor enthaltende oder im wesentlichen oder vollständig aus diesem bestehende Bereich kann insbesondere mit einem organischen halbleitenden Material und/oder einem anorganischen halbleitenden Material elektrisch stromleitend kontaktiert sein, beispielsweise auf einem derartigen Substrat angeordnet sein.

Vorzugsweise werden die genannten elektronenarmen Übergangsmetallkomplexverbindungen erfindungsgemäß als p-Dotandenprecursor bzw. p-Dotanden eingesetzt, z.B. in einem Verhältnis ≤ 1 : 1 oder ≤ 1 : 2. Mittels der erfindungsgemäß als Precursor bzw. p-Dotanden eingesetzten elektronenarmen Verbindungen können beispielsweise bei der Verwendung von ZnPc, Spiro-TTB odr α-NPD als Matrix halbleitende Schichten mit Leitfähigkeiten bei Raumtemperatur in dem Bereich von 10⁻⁵ S/cm oder höher erzielt werden, beispielsweise von 10⁻³ S/cm oder höher, beispielsweise von 10⁻¹ S/cm. Bei der Verwendung von Phthalocyanin-Zink als Matrix wurde eine Leitfähigkeit von höher 10⁻⁸ S/cm erzielt, beispielsweise 10⁻⁶ S/cm. Bisher war es nicht möglich, diese Matrix mit organischen Akzeptoren zu dotieren, da das Reduktionspotential der Matrix zu gering ist. Die Leitfähigkeit von undotiertem Phthalocyanin-Zink beträgt hingegen maximal 10⁻¹⁰ S/cm.

Es versteht sich, dass die Schicht oder das Gebilde mit den Dotanden jeweils ein oder mehrere verschiedene derartige elektronenarme Übergangsmetallkomplexverbindungen enthalten kann.

### Elektronisches Bauelement

Unter Verwendung der beschriebenen Verbindungen zur Herstellung p-dotierter organischer halbleitender Materialien, die insbesondere in Form von Schichten oder elektrischen Leitungspfaden angeordnet sein können, können eine Vielzahl elektronischer Bauelemente oder diese enthaltende Einrichtungen mit einer p-dotierten organischen Halbleiterschicht hergestellt werden. Im Sinne der Erfindung werden von dem Begriff "elektronische Bauelemente" auch optoelektronische Bauelemente mit umfasst. Durch die beschriebenen neuen Verbindungen können die elektronischen Eigenschaften eines elektronisch funktionell wirksamen Bereichs des Bauelementes, wie dessen elektrische Leitfähigkeit, lichtemittierende Eigenschaften oder dergleichen, vorteilhaft verändert werden. So kann die Leitfähigkeit der dotierten Schichten verbessert und/oder die Verbesserung der Ladungsträgerinjektion von Kontakten in die dotierte Schicht erreicht werden.

Die Erfindung umfasst insbesondere organische lichtemittierende Dioden (OLED), organische Solarzellen, Feldeffekt-Transistoren organische Dioden, insbesondere solche mit hohem Gleichrichtungsverhältnis wie 10³-10⁷,vorzugsweise 10⁴-10⁷ oder 10⁵-10⁷, und organische Feldeffekttransistoren, die mittels der elektronenarmen Übergangsmetallkomplexverbindungen hergestellt sind.

In dem elektronischen Bauelement kann eine p-dotierte Schicht auf Basis eines organischen Matrixmaterials beispielsweise in folgenden Schichtstrukturen vorliegen, wobei vorzugsweise die Basismaterialien oder Matrixmaterialien der einzelnen Schichten jeweils organisch sind:
p-i-n: p-dotierter Halbleiter-Isolator-n-dotierter Halbleiter,
n-i-p: n-dotierter Halbleiter-Isolator-p-dotierter Halbleiter.

"i" ist wiederum eine undotierte Schicht, "p" ist eine p-dotierte Schicht. Die Kontaktmaterialien sind hier löcherinjizierend, wobei p-seitig beispielsweise eine Schicht oder ein Kontakt aus ITO oder Au vorgesehen sein kann, oder elektroneninjizierend, wobei n-seitig eine Schicht oder ein Kontakt aus ITO, A1 oder Ag vorgesehen sein kann.

In obigen Strukturen kann im Bedarfsfall auch die i-Schicht ausgelassen werden, wodurch Schichtenabfolgen mit p-n oder n-p-Übergängen erhalten werden können.

Die Verwendung der beschriebenen Verbindungen ist jedoch auf die oben genannten Ausführungsbeispiele nicht beschränkt, insbesondere können die Schichtstrukturen durch Einführung zusätzlicher geeigneter Schichten ergänzt bzw. modifiziert werden. Insbesondere können jeweils OLEDs mit derartigen Schichtabfolgen, insbesondere mit pin- oder mit einer dazu inversen Struktur, mit den beschriebenen Verbindungen aufgebaut werden.

Mit Hilfe der beschriebenen p-Dotandenprecursom bzw. p-Dotanden können insbesondere organische Dioden vom Typ Metall-Isolator-p-dotierte Halbleiter (min) oder auch gegebenenfalls vom pin-Typ hergestellt werden, beispielsweise auf der Basis von Phthalozyaninzink. Diese Dioden zeigen ein Rektifizierungsverhältnis von 10⁵ und höher. Ferner können unter Verwendung der erfindungsgemäßen Precursor bzw. Dotanden elektronische Bauelemente mit p-n-Übergängen erzeugt werden, wobei für die p- und die n-dotierte Seite jeweils dasselbe Halbleitermaterial verwendet wird (Homo-p-n-Übergang), und wobei für das p-dotierte Halbleitermaterial eine beschriebene elektronenarme Übergangsmetallkomplexverbindung eingesetzt wird.

Die elektronenarmen Übergangsmetallkomplexverbindungen können erfindungsgemäß in den elektronischen Bauelementen aber auch in Schichten, Leitfähigkeitspfaden, Punktkontakten oder dergleichen eingesetzt werden, wenn diese gegenüber einer anderen Komponente überwiegen, beispielsweise als Injektionsschicht in reiner oder im wesentlichen in reiner Form.

Weitere Aufgaben und Vorteile der vorliegenden Erfindung werden nun anschaulich anhand der folgenden Beispiele beschrieben, die lediglich veranschaulichend und nicht als den Umfang der Erfindung begrenzend zu betrachten sind.

### Dotierbeispiele

Norbornadien-bis-cis-(1,2-perfluormethylen-1,2-dithiolato)nickel-Addukt (Verbindung 3) wird in 5%-iger Konzentration mit Spiro-TTB zusammen verdampft. Die gemessene Leitfähigkeit in der Schicht nach Belichtung mit einer Xenonlampe beträgt 5,1 x 10⁻⁵ S/cm, während die Leitfähigkeit von reinem Spiro- TTB 3 x 10⁻¹⁰ S/cm beträgt.

Norbornadien-bis-cis-(1,2-perfluormethylen-1,2-dithiolato)palladium-Addukt wird in 5%iger Konzentration mit Spiro-TTB zusammen verdampft. Die gemessene Leitfähigkeit in der Schicht nach Belichtung mit einer Xenon-Lampe beträgt 4,2 x 10⁻⁵S/cm.

Bis(1,2-di(trifluormethyl)-1,2-ethen-dithiolato)cobalt(0)-Norbornadien-Addukt wird in 10%iger Konzentration mit Spiro-TTB zusammen verdampft. Die gemessene Leitfähigkeit in der Schicht nach Belichtung mit einer Xenonlampe beträgt 1x10⁻⁶ S/cm.

Bis(1-heptafluor-iso-propyl-1,2-ethen-dithiolato)nickel(0)-Norbornadien-Addukt wird in 10%iger Konzentration mit Spiro-TTB zusammen verdampft. Die gemessene Leitfähigkeit in der Schicht nach Belichtung mit einer Xenonlampe beträgt 6,3 x 10⁻⁷ S/cm.

Unter Verwendung der besehriebenen Verbindungen zur Herstellung organischer halbleitender Materialien, die insbesondere in Form von Schichten oder elektrischen Leitungsfaden angeordnet sein können, kann eine Vielzahl elektronischer oder optoelektronischer Bauelemente hergestellt werden. Durch die beschriebenen Verbindungen können die elektronischen Eigenschaften eines elektronisch funktionell wirksamen Bereichs des Bauelements, wie dessen elektrische Leitfähigkeit, lichtemittierende Eigenschaften oder dergleichen, vorteilhaft verändert werden. So kann die Leitfähigkeit der dotierten Schichten verbessert und/oder die Verbesserung der Ladungsträgerinjektion von Kontakten in die dotierte Schicht erreicht werden.

Falls nicht anders vermerkt, wird als Dotand ein elektrischer Dotand verstanden, auch Redox-Dotand genannt, der die vorliegend beschriebenen Eigenschaften hat.

Die in der vorstehenden Beschreibung und in den Ansprüchen offenbarten Merkmale können sowohl einzeln als auch in jeder beliebigen Kombination Material zur Verwirklichung der Erfindung in ihren unterschiedlichsten Ausführungsformen darstellen.

## Patentansprüche

1. Übergangsmetallkomplex, der die folgenden Strukturen (4), (5) (6) oder (7) aufweist 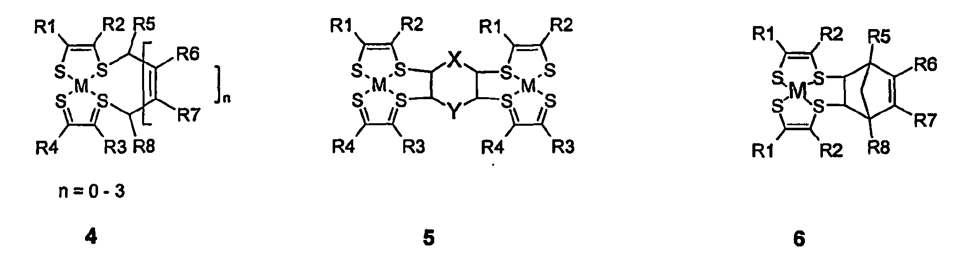 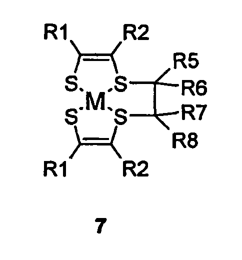 wobei M ein Übergangsmetall ist, das ausgewählt ist aus Cu, Au, Co, Zn, Cd und Hg;
R1-R4 unabhängig ausgewählt sind aus Wasserstoff; Halogen; Pseudohalogen; CN; NO₂; COR mit R = Alkyl, Alkenyl, Aryl oder Heteroaryl; geradkettigem, verzweigtem oder cyclischem, substituiertem oder unsubstituiertem Alkyl und Alkenyl; und substituiertem oder unsubstituiertem Aryl, Diaryl, Triaryl, Heteroaryl, Diheteroaryl oder Triheteroaryl
R5-R8 unabhängig ausgewählt sind aus Wasserstoff Alkyl, Alkenyl, Hetero-alkyl, Aryl, Heteroaryl, oder R5, R6, R7 und/oder R8 über eine oder mehrere Brücken miteinander verbunden sind; und
X und Y unabhängig voneinander ausgewählt sind aus O, S, NR, Alkyl, Alkenyl, Heteroalkyl, Heteroalkenyl, Aryl, Heteroaryl, wobei auch eines von X und Y nicht vorhanden sein kann zur Ausbildung einer einfach verbrückten Struktur (5).

2. Übergangsmetallkomplex nach Anspruch 1, **dadurch gekennzeichnet, dass** R1-R4 unabhängig ausgewählt sind aus geradkettigem, verzweigtem oder cyclischem, substituiertem oder unsubstituiertem Alkyl und Alkenyl, vorzugsweise perhalogeniertem Alkyl.

3. Übergangsmetallkomplex nach einem der vorangehenden Ansprüche, **dadurch gekennzeichnet, dass** R5-R8 unabhängig ausgewählt sind aus geradkettigem, verzweigtem, cyclischem, substituiertem oder unsubstituiertem Alkyl, Alkenyl, Heteroaryl, substituiertem und unsubstituiertem Aryl und Heteroaryl, Diaryl, Diheteroaryl, Triaryl und Triheteroaryl.

4. Verwendung eines Übergangsmetallkomplexes als Dotandenprecursor zur Dotierung und Photodotierung eines organischen halbleitenden Matrixmaterials, als Blockermaterial, als Ladungsinjektionsschicht, als Elektrodenmaterial, als Transportschicht, als Matrixmaterial selbst oder als Speichermaterial in elektronischen oder optoelektronischen Bauelementen, wobei der Übergangsmetallkomplex die folgenden Strukturen (4), (5), (6) oder (7) aufweist 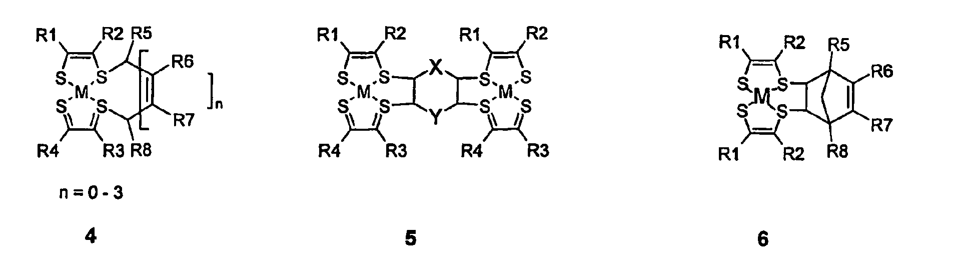 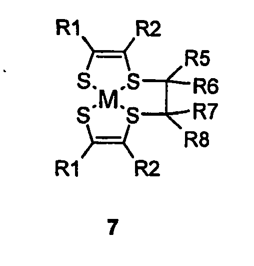 wobei M ein Übergangsmetall ist, das ausgewählt ist aus Ni, Pd, Pt, Cu, Au, Co, Zn, Cd und Hg;
R1-R4 unabhängig ausgewählt sind aus Wasserstoff, Halogen, Pseudohalogen, CN, NO₂ COR mit R = Alkyl, Alkenyl, Aryl oder Heteroaryl; geradkettigem, verzweigtem oder cyclischem, substituiertem oder unsubstituiertem Alkyl und Alkenyl; und substituiertem oder unsubstituiertem Aryl, Diaryl, Triaryl, Heteroaryl, Diheteroaryl oder Triheteroaryl;
R5-R8 unabhängig ausgewählt sind aus Wasserstoff Alkyl, Alkenyl, Hetero-alkyl, Aryl, Heteroaryl, oder R5, R6, R7 und/oder R8 über eine oder mehrere Brücken miteinander verbunden sind; und
X und Y unabhängig voneinander ausgewählt sind aus O, S, NR, Alkyl, Alkenyl, Heteroalkyl, Heteroalkenyl, Aryl, Heteroaryl, wobei auch eines von X und Y nicht vorhanden sein kann zur Ausbildung einer einfach verbrückten Struktur (5).

5. Verwendung nach Anspruch 4, **dadurch gekennzeichnet, dass** R1-R4 unabhängig ausgewählt sind aus geradkettigem, verzweigtem oder cyclischem, substituiertem oder unsubstituiertem Alkyl und Alkenyl, vorzugsweise perhalogeniertem Alkyl.

6. Organisches halbleitendes Material enthaltend zumindest eine organische Matrixverbindung und einen Dotandenprecursor, **dadurch gekennzeichnet, dass** als Dotandprecursor ein Übergangsmetallkomplex nach Anspruch 4 verwendet wird.

7. Organisches halbleitendes Material nach Anspruch 6, **dadurch gekennzeichnet, dass** das molare Dotierungsverhältnis von Dotandprecursor zum Matrixmolekül bzw. das Dotierungsverhältnis von Dotandprecursor zu monomeren Einheiten eines polymeren Matrixmoleküls zwischen 20:1 und 1:100000, bevorzugt 10:1 und 1:1000, besonders bevorzugt 1:1 1 und 1:100 beträgt.

8. Elektronisches oder optoelektronisches Bauelement mit einem elektronisch funktionell wirksamen Bereich, **dadurch gekennzeichnet, dass** als elektronisch wirksamer Bereich zumindest ein Übergangsmetallkomplex nach Anspruch 4 verwendet wird.

9. Elektronisches oder optoelektronisches Bauelement nach Anspruch 8, **dadurch gekennzeichnet, dass** der elektronisch wirksame Bereich ein organisches halbleitendes Material aufweist, welches mit zumindest einem Übergangsmetallkomplex nach Anspruch 4 dotiert ist.

10. Elektronisches oder optoelektronisches Bauelement nach Anspruch 8 oder 9 in Form einer organischen licht-emittierenden Diode, einer organischen Laserdiode, einer organischen Solarzelle, eines organischen Transistors, eines organischen Speicherbausteins, einer organischen Gleichrichterdiode, einer Photovoltaikzelle oder eines Photoleiters.

## Claims

1. A transition metal complex having the following structures (4), (5) (6) or (7) : 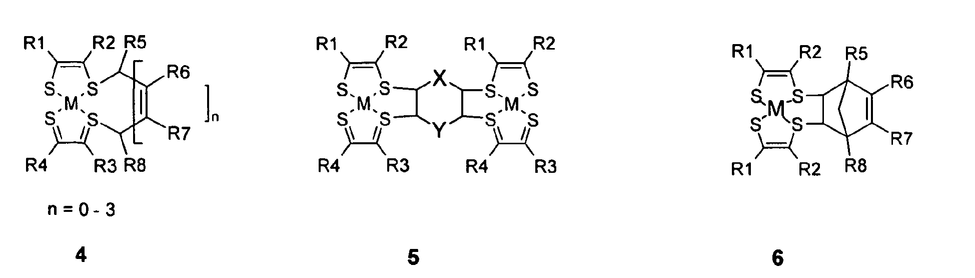 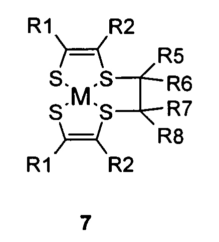 where M is a transition metal selected from Cu, Au, Co, Zn, Cd and Hg;
R1-R4 are selected independently from hydrogen; halogen; pseudohalogen; CN; NO₂; COR, where R = alkyl, alkenyl, aryl or heteroaryl; linear branched or cyclic, substituted or unsubstituted alkyl and alkenyl; and substituted or unsubstituted aryl, diaryl, triaryl, heteroaryl, diheteroaryl or triheteroaryl;
R5-R8 are selected independently from hydrogen, alkyl, alkenyl, heteroalkyl, aryl, heteroaryl, or R5, R6, R7 and/or R8 is/are connected to one another via one or more bridges; and
X and Y, independently of one another, are selected from O, S, NR, alkyl, alkenyl, heteroalkyl, heteroalkenyl, aryl, heteroaryl, wherein one of X and Y cannot not be present to form a structure (5) having a single bridge.

2. The transition metal complex according to Claim 1, **characterized in that** R1-R4 are selected independently from linear, branched or cyclic, substituted or unsubstituted alkyl and alkenyl, preferably perhalogenated alkyl.

3. The transition metal complex according to any one of the preceding claims, **characterized in that** R5-R8 are selected independently from linear, branched, cyclic, substituted or unsubstituted alkyl, alkenyl, heteroalkyl, substituted and unsubstituted aryl and heteroaryl, diaryl, diheteroaryl, triaryl and triheteroaryl.

4. Use of a transition metal complex as a dopant precursor for doping and photodoping of an organic semiconducting matrix material, as a blocking material, as a charge injection layer, as an electrode material, as a transport layer, as a matrix material itself or as a memory material in electronic or optoelectronic components, wherein the transition metal complex has the following structures (4), (5), (6) or (7): 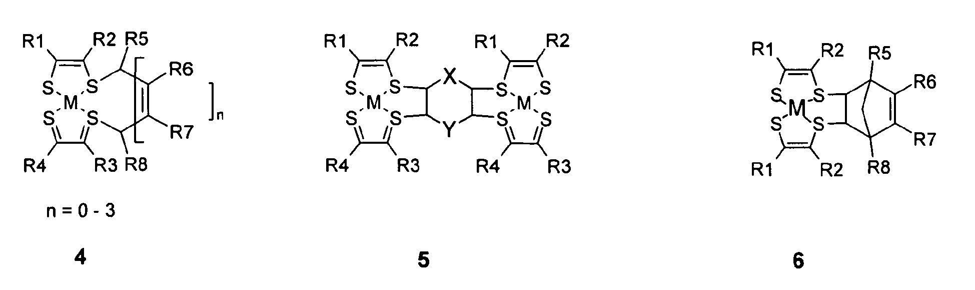 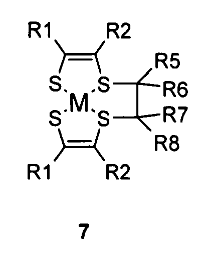 where M is a transition metal which is selected from Ni, Pd, Pt, Cu, Au, Co, Zn, Cd and Hg;
R1-R4 are selected independently from hydrogen, halogen, pseudohalogen, CN, NO₂, COR, where R = alkyl, alkenyl, aryl or heteroaryl; linear, branched or cyclic, substituted or unsubstituted alkyl and alkenyl; and substituted or unsubstituted aryl, diaryl, triaryl, heteroaryl, diheteroaryl or triheteroaryl;
R5-R8 are selected independently from hydrogen, alkyl, alkenyl, heteroalkyl, aryl, heteroaryl, or R5, R6, R7 and/or R8 are linked to one another via one or more bridges; and
X and Y, independently of one another, are selected from O, S, NR, alkyl, alkenyl, heteroalkyl, heteroalkenyl, aryl, heteroaryl, wherein one of X and Y cannot be present to form a structure (5) having a single bridge.

5. Use according to Claim 4, **characterized in that** R1-R4 are selected independently from linear, branched or cyclic, substituted or unsubstituted alkyl and alkenyl, preferably perhalogenated alkyl.

6. An organic semiconductor material containing at least one organic matrix compound and a dopant precursor, **characterized in that** a transition metal complex according to Claim 4 is used as the dopant precursor.

7. The organic semiconducting material according to Claim 6, **characterized in that** the molar doping ratio of dopant precursor to matrix molecule and/or the doping ratio of dopant precursor to monomeric units of a polymer matrix molecule is/are between 20:1 and 1:100,000, preferably 10:1 and 1:1000, especially preferably 1:1 and 1:100.

8. An electronic or optoelectronic component having an electronically functional active range, **characterized in that** at least one transition metal complex according to Claim 4 is used as the electronically active range.

9. The electronic or optoelectronic component according to Claim 8, **characterized in that** the electronically active range as an organic semiconducting material which is doped with at least one transition metal complex according to Claim 4.

10. The electronic or optoelectronic component according to Claim 8 or 9 in the form of an organic light-emitting diode, an organic laser diode, an organic solar cell, an organic transistor, an organic memory module, an organic rectifier diode, a photovoltaic cell or a photoconductor.

## Revendications

1. Complexe de métal de transition présentant les structures (4), (5) (6) ou (7) suivantes 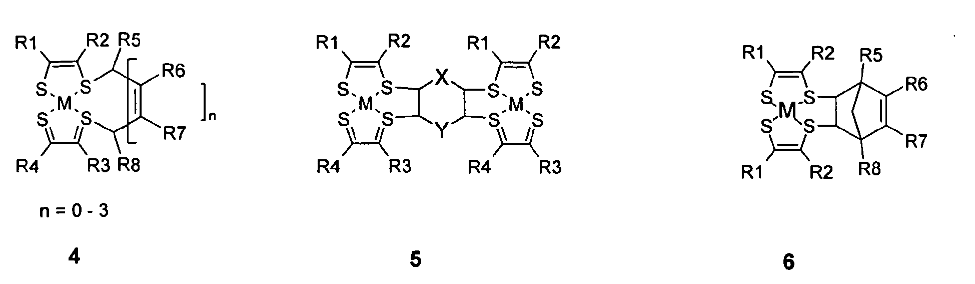 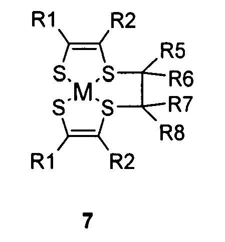 dans lesquelles M est un métal de transition choisi parmi Cu, Au, Co, Zn, Cd et Hg;
R1-R4 étant indépendamment choisis parmi l'hydrogène ; les halogènes ; les pseudohalogènes ; CN ; NO₂; COR avec R = alkyle, alcényle, aryle ou hétéroaryle ; les alkyles et alcényles linaires, ramifiés ou cycliques pourvus de substituants ou non ; et les aryles, diaryles, triaryles, hétéroaryles, dihétéroaryles ou trihétéroaryles pourvus de substituants ou non
R5-R8 étant indépendamment choisis parmi l'hydrogène, les alkyles, alcényles, hétéroalkyles, aryles, hétéroaryles, ou R5, R6, R7 et/ou R8 étant reliés entre eux par une ou plusieurs liaisons pontées ; et
X et Y étant indépendamment l'un de l'autre choisis parmi O, S, NR, les alkyles, alcényles, hétéroalkyles, hétéroalcényles, aryles, hétéroaryles, l'un des motifs X et Y pouvant être absent pour ainsi former une structure avec liaison pontée simple (5).

2. Complexe de métal de transition selon la revendication 1, **caractérisé en ce que** R1-R4 sont indépendamment choisis parmi les alkyles et alcényles linaires, ramifiés ou cycliques pourvus de substituants ou non, de préférence parmi les alkyles perhalogénés.

3. Complexe de métal de transition selon l'une des revendications précédentes, **caractérisé en ce que** R5-R8 sont indépendamment choisis parmi les alkyles, alcényles, hétéroalkyles linaires, ramifiés ou cycliques pourvus de substituants ou non, les aryles pourvus de substituants ou non et les hétéroaryles, diaryles, dihétéroaryles, triaryles et les trihétéroaryles.

4. Utilisation d'un complexe de métal de transition en tant que précurseur de dopant destiné au dopage et photodopage d'un matériau de matrice organique semi-conducteur, en tant que matériau de blocage, en tant que couche d'injection de charges, en tant que matériau d'électrode, en tant que couche de transport, en tant que matériau de matrice proprement dit, ou en tant que matériau de mémoire dans des composants électronique ou optoélectronique, ledit complexe de métal de transition présentant les structures (4), (5), (6) ou (7) suivantes 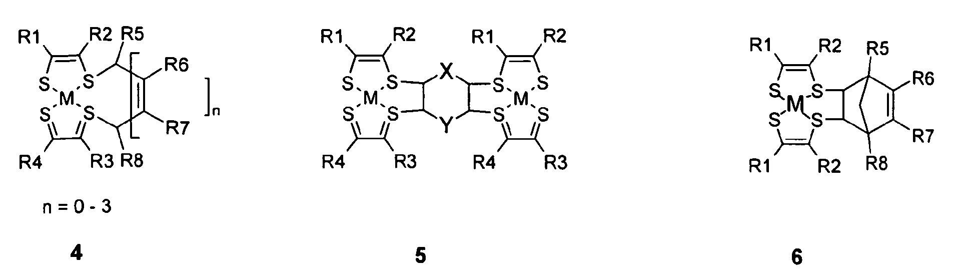 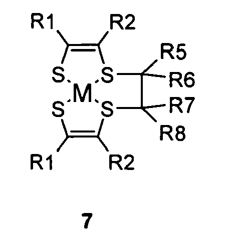 dans lesquelles M est un métal de transition choisi parmi Ni, Pd, Pt, Cu, Au, Co, Zn, Cd et Hg ;
R1-R4 étant indépendamment choisis parmi l'hydrogène, les halogènes, les pseudohalogènes, CN, NO₂, COR avec R = alkyle, alcényle, aryle ou hétéroaryle ; les alkyles et alcényles linaires, ramifiés ou cycliques pourvus de substituants ou non ; et les aryles, diaryles, triaryles, hétéroaryles, dihétéroaryles ou trihétéroaryles pourvus de substituants ou non ;
R5-R8 étant indépendamment choisis parmi l'hydrogène, les alkyles, alcényles, hétéroalkyles, aryles, hétéroaryles, ou R5, R6, R7 et/ou R8 étant reliés entre eux par une ou plusieurs liaisons pontées ; et
X et Y étant indépendamment l'un de l'autre choisis parmi O, S, NR, les alkyles, alcényles, hétéroalkyles, hétéroalcényles, aryles, hétéroaryles, l'un des motifs X et Y pouvant être absent pour ainsi former une structure avec liaison pontée simple (5).

5. Utilisation selon la revendication 4, **caractérisé en ce que** R1-R4 sont indépendamment choisis parmi les alkyles et alcényles linaires, ramifiés ou cycliques pourvus de substituants ou non, de préférence parmi les alkyles perhalogénés.

6. Matériau organique semi-conducteur, contenant au moins un composé de matrice organique et un précurseur de dopant, **caractérisé en ce que** l'on utilise, en tant précurseur de dopant, un complexe de métal de transition selon la revendication 4.

7. Matériau organique semi-conducteur selon la revendication 6, **caractérisé en ce que** le rapport molaire de dopage établi entre le précurseur de dopant et la molécule de matrice ou, le cas échéant, le rapport de dopage établi entre le précurseur de dopant et des unités monomères d'une molécule de matrice polymère, se situe entre 20 : 1 et 1 : 100 000, de préférence entre 10 : 1 et 1 : 1 000, avec une préférence particulière entre 1 : 1 et 1 : 100.

8. Composant électronique ou optoélectronique avec une partie présentant une fonctionnalité électronique, **caractérisé en ce que** l'on utilise, en tant que partie à effet électronique, au moins un complexe de métal de transition selon la revendication 4.

9. Composant électronique ou optoélectronique selon la revendication 8, **caractérisé en ce que** ladite partie à effet électronique comporte un matériau organique semi-conducteur qui est dopé avec au moins un complexe de métal de transition selon la revendication 4.

10. Composant électronique ou optoélectronique selon la revendication 8 ou 9, sous forme d'une diode électroluminescente organique, d'une diode laser organique, d'une cellule solaire organique, d'un transistor organique, d'un module de mémoire organique, d'une diode de redresseur organique, d'une cellule photovoltaïque ou d'une photorésistance.
